# EUROPÄISCHE PATENTSCHRIFT

(11) **EP 0 617 035 B1**
(45) Veröffentlichungstag und Bekanntmachung des Hinweises auf die Patenterteilung: **12.11.1997**
(21) Anmeldenummer: 94103448.0
(22) Anmeldetag: 07.03.1994
(51) Int. Cl.: C07D 471/04, C07D 209/86, A61K 31/40, A61K 31/44

(54) **Hetero-tricyclisch-substituierte Phenyl-cyclohexan-carbonsäurederivate**
Heterotricyclic substituted phenylcyclohexanecarboxylic acid derivatives
Dérivés de l'acide phényle-cyclohexane-carboxylique substitués par des groupes hétéro-tricycliques

(30) Priorität: 18.03.1993 DE 4308788
(43) Veröffentlichungstag der Anmeldung: 28.09.1994
(73) Patentinhaber: BAYER AG, 51368 Leverkusen (DE)
(72) Erfinder: Müller, Ulrich E., Dr., D-4211 Wuppertal (DE); Dressel, Jürgen, Dr., D-42477 Radevormwald (DE); Fey, Peter, Dr., D-42111 Wuppertal (DE); Hanko, Rudolf H., Dr., D-40237 Düsseldorf (DE); Hübsch, Walter, Dr., D-42113 Wuppertal (DE); Krämer, Thomas, Dr., D-42111 Wuppertal (DE); Müller-Gliemann, Matthias, Dr., D-42697 Solingen 10 (DE); Beuck, Martin, Dr., D-40699 Erkrath (DE); Kazda, Stanislav, Dr., D-42115 Wuppertal (DE); Wohlfeil, Stefan, Dr., D-40724 Hilden (DE); Knorr, Andreas, Dr., D-40699 Erkrath (DE); Stasch, Johannes-Peter, Dr., D-42651 Solingen (DE); Zaiss, Siegfried, Dr., D-42113 Wuppertal (DE)

(56) Entgegenhaltungen:
- EP-A- 0 217 737
- EP-A- 0 218 541
- EP-A- 0 249 301
- EP-A- 0 253 310
- EP-A- 0 324 377
- EP-A- 0 399 731
- EP-A- 0 399 732
- EP-A- 0 407 102
- EP-A- 0 497 516
- EP-A- 0 511 791
- US-A- 4 719 211
- US-A- 5 212 195
- EUR. J. MED. CHEM. - CHIMICA THERAPEUTICA, Bd.11, Nr.6, 1976 Seiten 493 - 499 M. LANGLOIS ET AL.

## Beschreibung

Die Erfindung betrifft hetero-tricyclisch-substituierte Phenyl-cyclohexan-carbonsäurederivate, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin 11 abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüberhinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Aus den Dokumenten EP-A-0 497 516, EP-A-0 511 791, US 4,719,211, EP-A-0 249 301, EP-A-0 218 541, EP-A-0 217 737 ist bekannt, daß Imidazolyl substituierte Benzyl-derivate, die im Molekül durch Carboxy-, Alkoxycarbonyl- oder Carboxamidosulfonylgruppen substituiert sind, Angiothensin-II-antagoistische Eigenschaften aufweisen.

Außerdem sind aus den Publikationen EP 407 102, EP 399 731, EP 399 732, EP 324 377 und EP 253 310 heterocyclische Verbindungen mit A II-antagonistischer Wirkung bekannt.

Die Erfindung betrifft jetzt hetero-tricyclisch-substituierte Phenyl-cyclohexancarbonsäurederivate der allgemeinen Formel (I) 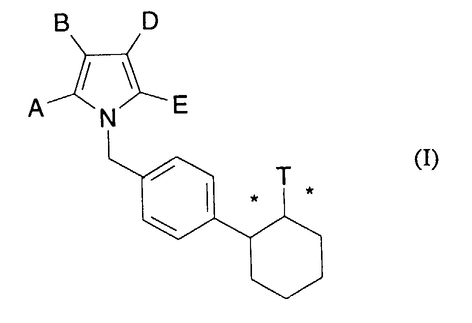 in welcher
- A und B: gemeinsam einen Rest der Formel 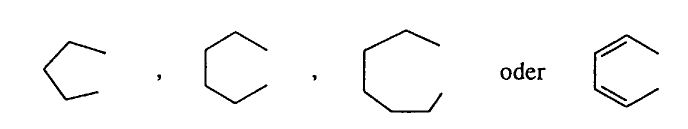 bilden,
- D und E: gemeinsam einen Rest der Formel 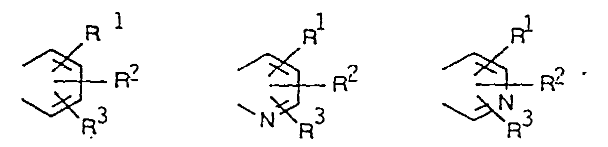 bilden, 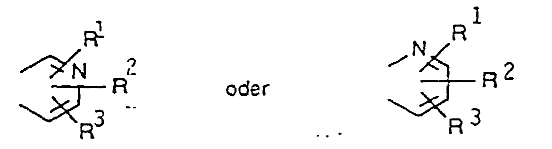 worin
R¹, R² und R³ gleich oder verschieden sind und Wasserstoff oder für Aryl mit 6 bis 10 Kohlenstoffatomen bedeuten, das gegebenenfalls durch Hydroxy, Halogen, Trifluormethyl oder durch geradkettiges oder verzweigtes Alkyl oder Alkoxy mit jeweils bis zu 6 Kohlenstoffatomen substiutiert ist, oder geradkettiges oder verzweigtes Alkyl oder Alkenyl mit jeweils bis zu 8 Kohlenstoffatomen bedeuten, oder Cycloalkyl mit 3 bis 8 Kohlenstoffatomen bedeuten, oder Halogen bedeuten,
- T: für einen Rest der Formel 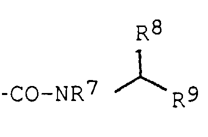 steht,
worin
- R⁷: Wasserstoff oder geradkettiges oder verzweigtes Alkyl mit bis zu 6 Kohlenstoffatomen beteutet,
- R⁸: Aryl mit 6 bis 10 Kohlenstoffatomen bedeutet, das gegebenenfalls bis zu 2-fach gleich oder verschieden durch Halogen, Hydroxy, geradkettiges oder verzweigtes Alkyl, Alkoxy oder Alkoxycarbonyl mit jeweils bis zu 6 Kohlenstoffatomen, Carboxy, Phenoxy oder C₃-C₆-Cycloalkyloxy substituiert ist,
- R⁹: eine Gruppe der Formel -CH₂-OR¹⁰, -CO₂R¹¹, -CO-NR¹²R¹³ oder Pyridyl bedeutet,
worin
- R¹⁰: Wasserstoff oder geradkettiges oder verzweigtes Alkyl mit bis zu 8 Kohlenstoffatomen bedeutet,
- R¹¹: Wasserstoff, geradkettiges oder verzweigtes alkyl mit bis zu 8 Kohlenstoffatomen, Phenyl oder Cycloalkyl mit 3 bis 6 Kohlenstoffatomen bedeutet,
- R¹² und R¹³: gleich oder verschieden sind und Wasserstoff, geradkettiges oder verzweigtes Alkyl mit bis zu 8 Kohlenstoffatomen oder Phenyl bedeuten,
gegebenenfalls in einer isomeren Form, und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der neuen hetero-tricyclisch substituierten Phenyl-cyclohexan-1-carbonsäuren und -carbonsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- A und B: gemeinsam einen Rest der Formel 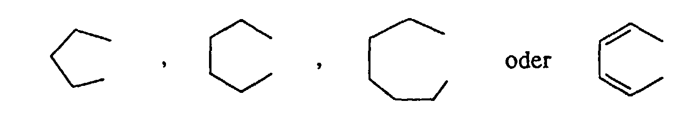 bilden,
- D und E: gemeinsam einen Rest der Forrnel 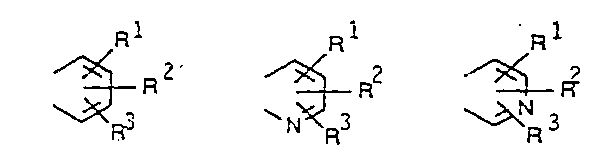 bilden, 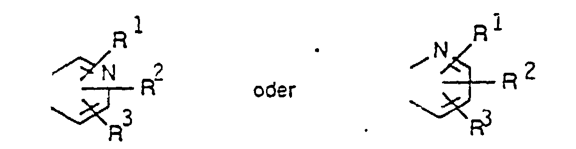 worin
R¹, R² und R³ gleich oder verschieden sind und Wasserstoff oder für Phenyl bedeuten, das gegebenenfalls durch Hydroxy, Fluor, Chlor, Brom, Trifluormethyl oder durch geradkettiges oder verzweigtes Alkyl oder Alkoxy mit jeweils bis zu 4 Kohlenstoffatomen substituiert ist, oder geradkettiges oder verzweigtes Alkyl oder Alkenyl mit jeweils bis zu 6 Kohlenstoffatomen bedeuten, oder Cyclopropyl, Cyclobutyl, Cyclopentyl, Cyclohexyl oder Cycloheptyl bedeuten, oder Fluor, Chlor oder Brom bedeuten,
- T: für einen Rest der Formel 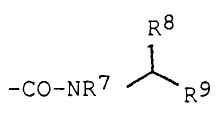 steht,
worin
- R⁷: Wasserstoff oder geradkettiges oder verzweigtes Alkyl mit bis zu 4 Kohlenstoffatomen bedeutet,
- R⁸: Phenyl bedeutet, das gegebenenfalls durch Fluor, Chlor, Brom, Hydroxy, geradkettiges oder verzweigtes Alkyl, Alkoxy oder Alkoxycarbonyl mit jeweils bis zu 4 Kohlenstoffatomen, Carboxy oder Phenoxy substituiert ist,
- R⁹: eine Gruppe der Formel -CH₂-OR¹⁰, -CO₂R¹¹, -CO-NR¹²R¹³ oder Pyridyl bedeutet,
worin
R¹⁰ Wasserstoff oder geradkettiges oder verzweigtes Alkyl mit bis zu 6 Kohlenstoffatomen bedeutet,
R¹¹ Wasserstoff, geradkettiges oder verzweigtes Alkyl mit bis zu 6 Kohlenstoffatomen, Phenyl, Cyclopropyl, Cyclopentyl oder Cyclohexyl bedeutet,
R¹² und R¹³ gleich oder verschieden sind und Wasserstoff, geradkettiges oder verzweigtes Alkyl mit bis zu 6 Kohlenstoffatomen, Cyclopropyl, Cylcopentyl, Cylcohexyl oder Phenyl bedeuten,
gegebenenfalls in einer isomeren Form, und deren Salze.

Besonders bevorzugt sind Verbindungen der allgemeinen Formel (I),
in welcher
- A und B: gemeinsam einen Rest der Formel 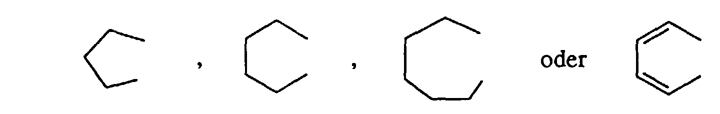 bilden,
- D und E: gemeinsam einen Rest der Formel 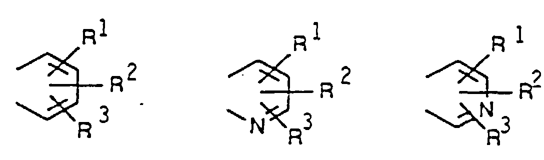 bilden, 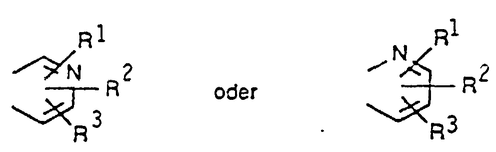 worin
R¹, R² und R³ gleich oder verschieden sind und Wasserstoff, Methyl, Fluor, Chlor oder Brom bedeuten,
- T: für einen Rest der Formel 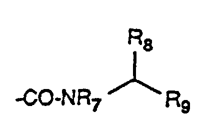 steht,
worin
- R⁷: Wasserstoff oder Methyl bedeutet,
- R⁸: Phenyl bedeutet, das gegebenenfalls durch Fluor, Chlor, Brom, Hydroxy, geradkettiges oder verzweigtes Alkyl, Alkoxy oder Alkoxycarbonyl mit jeweils bis zu 3 Kohlenstoffatomen, Carboxy oder Phenoxy substituiert ist,
- R⁹: eine Gruppe der Formel -CH₂-OR¹⁰, -CO₂-R¹¹, -CO-NR¹²R¹³ oder Pyridyl bedeutet,
worin
R¹⁰ Wasserstoff oder geradkettiges oder verzweigtes Alkyl mit bis zu 4 Kohlenstoffatomen bedeutet,
R¹¹ Wasserstoff, geradkettiges oder verzweigtes Alkyl mit bis zu 4 Kohlenstoffatomen, Phenyl, Cyclopropyl, Cyclopentyl oder Cyclohexyl bedeutet,
R¹² und R¹³ gleich oder verschieden sind und Wasserstoff, geradkettiges oder verzweigtes Alkyl mit bis zu 4 Kohlenstoffatomen, Cyclopropyl, Cyclopentyl, Cyclohexyl oder Phenyl bedeuten,
gegebenenfalls in einer isomeren Form, und deren Salze.

Besonders bevorzugt sind Verbindungen der allgemeinen Formel (I),
in welcher
- A und B: gemeinsam einen Rest der Formel 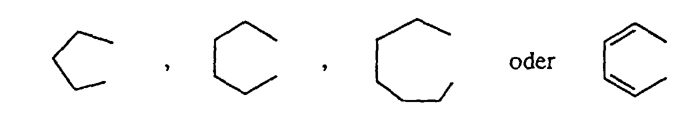 bilden,
- D und E: gemeinsam einen Rest der Formel 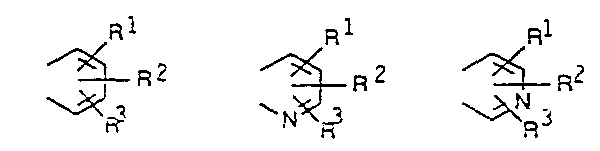 bilden, 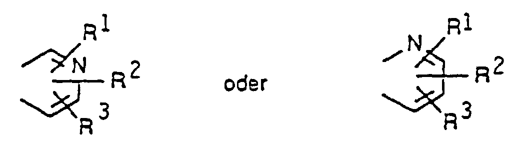 worin
R¹, R² und R³ gleich oder verschieden sind und für Wasserstoff, Brom oder Methyl stehen,
- T: für einen Rest der Formel 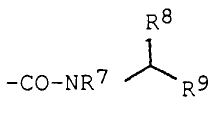 steht,
worin
R⁷ Wasserstoff bedeutet,
R⁸ Phenyl bedeutet,
R⁹ eine Gruppe -CH₂OH bedeutet,
gegebenenfalls in einer isomeren Form, und deren Salze.

Außerdem wurde ein Verfahren zur Herstellung der erfindungsgemäßen Verbindungen der allgemeinen Formel (I) gefunden, dadurch gekennzeichnet, daß man

Verbindungen der allgemeinen Formel (II) 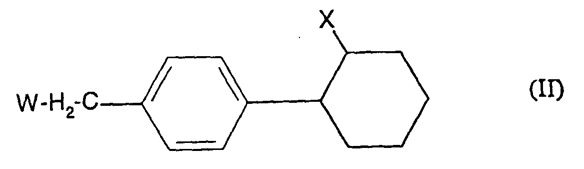 in welcher
- W: für eine typische Abgangsgruppe wie beispielsweise Chlor, Brom, Jod, Tosylat oder Mesylat, vorzugsweise für Brom steht
und
- X: für C₁-C₆-Alkoxycarbonyl steht,
zunächst mit Verbindungen der allgemeinen Formel (III) 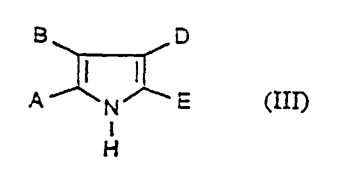 in welcher
A, B, D und E die oben angegebene Bedeutung haben,
in inerten Lösemitteln, gegebenenfalls in Anwesenheit einer Base und gegebenenfalls unter Schutzgasatmosphäre zu Verbindungen der allgemeinen Formel (IV) 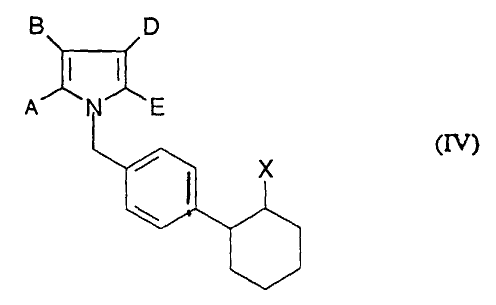 in welcher
A,B,D,E und X die oben angebebene Bedeutung haben,
umsetzt, und gegebenenfalls ausgehend von den entsprechenden Carbonsäuren, nach vorgeschalteter Hydrolyse und/oder Aktivierung, anschließend Aminen der allgemeinen Formel (Va) 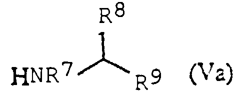 in welchen
R⁷, R⁸ und R⁹ die oben angegebene Bedeutung haben,
gegebenenfalls in Anwesenheit einer Base und/oder eines Hilfsmittels, beispielsweise eines Dehydratisierungsmittels, in inerten Lösemitteln amidiert,
und gegebenenfalls die Isomeren trennt, und im Fall der Herstellung der Salze mit einer entsprechenden Base oder Säure umsetzt.

Das erfindungsgemäße Verfahren kann durch folgendes Formelschema beispielhaft erläutert werden:

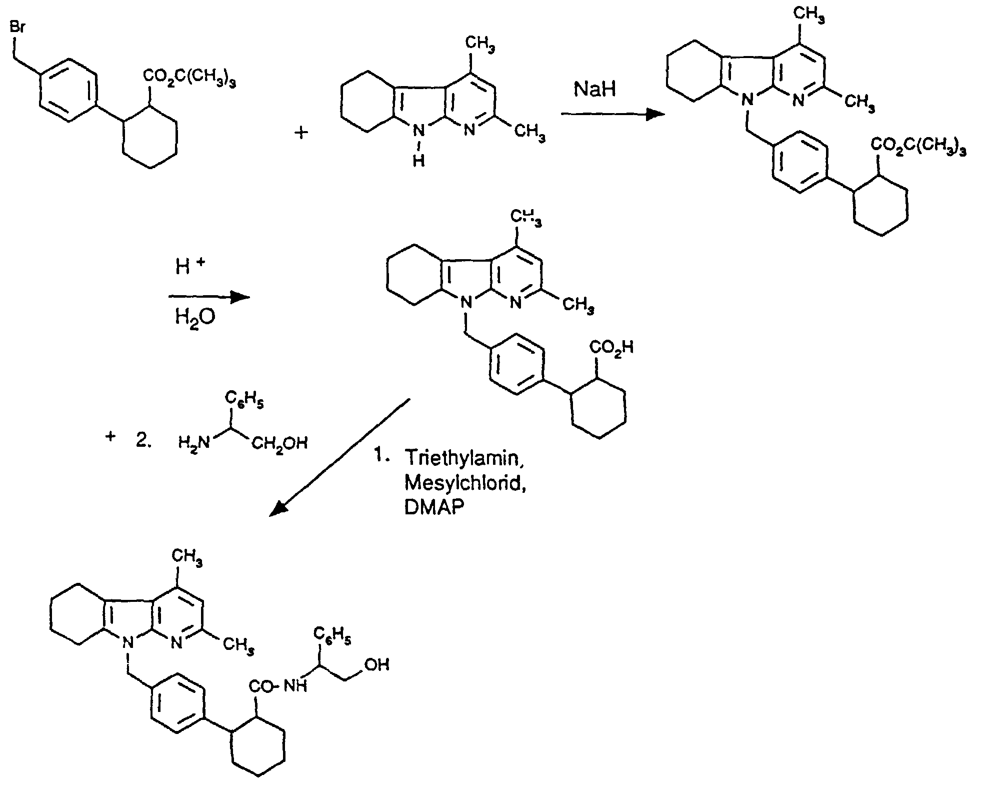

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphor säuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (III), ein.

Das erfindungsgemäße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumhydroxid eingesetzt.

Als Lösemittel eignen sich für die Hydrolyse Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Hydrolyse kann auch mit wäßrigen Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Hydrolyse wird im allgemeinen in einem Temperaturbereich von 0°C bis +100°C, bevorzugt von +20°C bis +80°C durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Hydrolyse wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Amidierung der Verbindungen der allgemeinen Formel (IV) erfolgt im allgemeinen in einem der oben aufgeführten Lösemitteln, vorzugsweise in Tetrahydrofuran oder Dichlormethan.

Die Amidierung kann gegebenenfalls über die aktivierte Stufe der Säurehalogenide [(IV) Y = Halogen], die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung erfolgt im allgemeinen in einem Temperaturbereich von -80°C bis +80°C, vorzugsweise von -30°C bis +30°C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der Verbindungen der allgemeinen Formel (IV) eingesetzt.

Als säurebindende Mittel für die Amidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren eingesetzt.

Die Cyclohexan-Verbindungen der allgemeinen Formel (II) sind größtenteils neu und können hergestellt werden, indem man Verbindungen der allgemeinen Formel (VI) 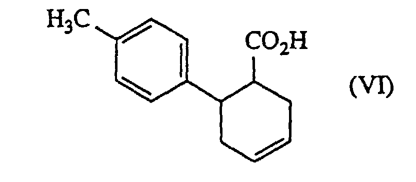 zunächst durch Hydrierung mit Palladium/C in einem der oben angegebenen Lösemitteln, vorzugsweise Methanol, in einer Wasserstoffatmosphäre in die Verbindungen der allgemeinen Formel (VII) 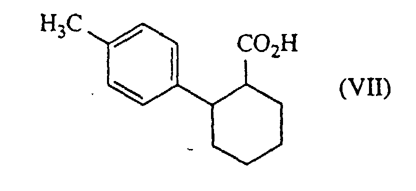 überführt,
in einem zweiten Schritt, im Fall, daß T ≠ Tetrazol, nach üblichen Methoden verestert,
und in einem letzten Schritt an der Methylengruppe eine Bromierung, gegebenenfalls in Anwesenheit eines Katalysators, durchführt.

Die Reduktion der Doppelbindung erfolgt in einem Temperaturbereich von 0°C bis +40°C, vorzugsweise bei +20°C und einem Druck von 1 bar.

Die Veresterung erfolgt in einem der oben angegebenen Lösemitteln, vorzugsweise Toluol und Tetrahydrofuran, nach der oben bereits beschriebenen vorgeschalteten Aktivierung der entsprechenden Carbonäsure, vorzugsweise über die Carbonsäurechloride, und nachträglicher Umsetzung mit dem entsprechenden Alkoholaten, in einem Temperaturbereich von 0°C bis +60°C, vorzugsweise bei +10°C bis +35°C und Normaldruck.

Die Bromierung wird im allgemeinen in einem Temperaturbereich von +40°C bis 100°C, vorzugsweise von +60°C bis +90°C und Normaldruck durchgeführt. Sie erfolgt in einem der oben angegebenen Lösemitteln, vorzugsweise mit Tetrachlorkohlenstoff, und mit N-Bromsuccinimid.

Als Starter (Katalysator) für die Bromierung eignen sich beispielsweise Azobisisobutyronitril, Dibenzoylperoxid, vorzugsweise Azobisisobutyronitril, wobei der Starter in einer Menge von 0,01 mol bis 0,1 mol, bevorzugt von 0,01 mol bis 0,05 mol, bezogen auf 1 mol der Verbindung der allgemeinen Formel (VII), eingesetzt wird.

Die Verbindungen der allgemeinen Formel (VI) sind ebenfalls neu und können beispielsweise hergestellt werden, indem man Verbindungen der allgemeinen Formel (VIII) 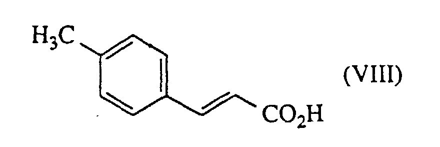 in einem der oben angegebenen Lösemittel, vorzugsweise Toluol, mit 1,3-Butadien in Anwesenheit von Hydrochinon, in einem Temperaturbereich von +180°C bis +230°C, vorzugsweise bei 200°C und einem Druck von ca. 20 bar umsetzt [vgl. hierzu Eur. J. Med. Chem. 11, 493 (1976)].

Die Verbindungen der allgemeinen Formel (VIII) sind an sich bekannt oder können nach üblichen Methoden hergestellt werden.

Die Verbindungen der allgemeinen Formeln (IV) und (VII) sind neu und können beispielsweise nach den oben beschriebenen Verfahren hergestellt werden.

Ebenso sind die Verbindungen der allgemeinen Formel (III) an sich bekannt oder können nach üblicher Methode hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretions-stimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

### Agonisten und ihre Standardkonzentrationen Applikationsvolumen pro Einzelgabe = 100 µl):

| | | |
|---|---|---|
| KCl | 22,7;32,7;42,7;52,7 | mmol/l |
| 1 Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt Bei dieser Hypertonieform ist die Plasmareninaktivität in den ersten sechs Wochen nach dem Eingriff erhöht
Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0.25 ml durchgeführt, das im einzelnen die partiell gereinigten Membranen (50 - 80 µg), ³H-Angiotensin II (3-5 nM), Test-Pufferlösung (50 mM Tris, pH 7,2, 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Ki: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

**Tabelle A:**

| Bsp.-Nr. | IC₅₀ [nM] |
|---|---|
| 27 | 580 |
| 30 | 0,1 |
| 32 | 2,0 |
| 50 | 0,9 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Laufmittel:

- A: Petrolether: Essigester = 1:1
- B: Dichlormethan: Methanol = 50:1
- C: Petrolether: Essigester = 5:1
- D: Dichlormethan
- E: Petrolether : Dichlormethan = 3:1
- F: Dichlormethan : Methanol = 20:1
- G: Dichlormethan : Methanol = 10:1
- H: Dichlormethan : Essigester = 1:1
- I: Dichlormethan : Essigester = 2:1
- J: Dichlormethan: Methanol: konz. wäß. Ammoniak = 200:20:1
- K: Petrolether: Essigester = 4:1
- L: Dichlormethan : Essigester = 10:1
- M: Petrolether : Essigester = 10:1
- N: Dichlormethan: Methanol = 7:1

### Ausgangsverbindungen

### Beispiel I

trans-6-(4-Tolyl)-cyclohex-3-en-1-carbonsäure 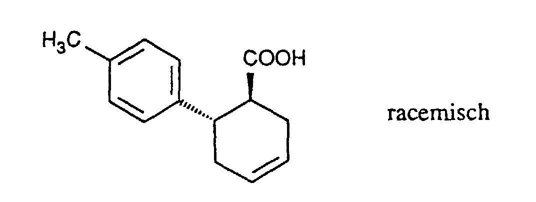 275 g (1,695mol) 3-E-(4-Tolyl)acrylsäure (käuflich) wird nach einem bekannten Verfahren (Eur.J. Med. Chem. 11, 493 (1976)) in 480 ml Toluol mit 580 ml 1,3-Butadien (in kondensierter Form abgemessen) unter Zugabe von 3 g Hydrochinon 22 h bei ca 200°C und ca 20 bar umgesetzt Das Rohgemisch wird mit Toluol verdünnt und mit 0,5 M wäßriger Natronlauge extrahiert. Darauf werden die wäßrigen Phasen mit 1 M Salzsäure angesäuert und mit Ether extrahiert. Die etherischen Lösungen werden mit Natriumsulfat getrocknet, eingedampft und wiederum in Toluol gelöst. Nach 15 minütigem Kochen mit 5g Aktivkohle saugt man heiß ab und dampft das Lösemittel bis auf ca. 120 - 160 ml ab; bei 0 - 4°C kristallisieren 124 g (573 mmol) Produkt aus. Das Filtrat wird etwas weiter eingeengt und zum Nachkristallisieren wieder abgekühlt. Bei Wiederholung dieses Vorgangs fallen insgesamt weitere 42 g (194 mmol) Produkt an.
R_{f} = 0,39 (Dichlormethan: Methanol = 10:1)

### Beispiel II

trans-2-(4-Tolyl)-cyclohexan-1-carbonsäure 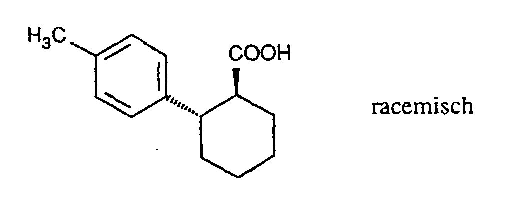 155 g (717 mmol) der Verbindung aus Beispiel I werden in 1 l Methanol gelöst und an 10 g Palladium (10% auf Tierkohle) bei 20°C und Wassestoffatmosphäre von ca 1 bar umgestzt. Nach einer Reaktionszeit von insgesamt 16 h wird der Katalysator abfiltriert und das Lösemittel - zuletzt im Vakuum - abgedampft
Ausbeute: 153 g (701 mmol)
R_{f}= 0,38 (Dichlormethan: Methanol = 10:1)

### Beispiel III

trans-2-(4-Tolyl)-cyclohexan-1-carbonsäure-tert.butylester

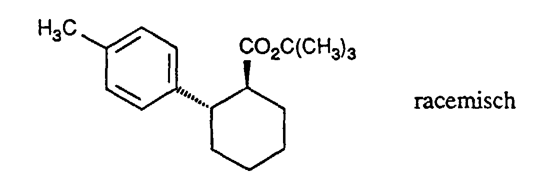

### Methode A:

45,8 g (184 mmol) der Verbindung aus Beispiel II werden in 600 ml Toluol gelöst und unter Rückfluß mit 49,5 ml (387 mmol) Oxalylchlorid zur Reaktion gebracht. Nach 2 h dampft man das Lösemittel mit überschüssigem Reagenz ab, dazu muß das rohe Carbonsäurechlorid gegebenenfalls wiederholt in Toluol aufgenommen und noch einmal abrotiert werden. Das so gewonnene Produkt wird in 500 ml Tetrahydrofuran gelöst, mit 24,8 g (221 mmol) Kalium-tert.butanolat bei 0°C verrührt und 20 h nachgerührt (bei 20°C). Darauf werden Wasser und Ether zugesetzt und mehrfach extrahiert. Die organische Phase wird mit Natriumsulfat getrocknet, eingedampft und der Rückstand an Kieselgel 60 (Merck, Petrolether : Essigester = 20:1) chromatographisch gereinigt.
Ausbeute: 39,6 g (130 mmol)
R_{f} = 0,47 (Petrolether: Essigester = 10:1)

### Methode B

20,0 g (91,6 mmol) der Verbindung aus Beispiel II werden in 7 ml konz. Schwefelsäure in 100 ml Ether suspendiert und bei -30°C mit 80 ml (713 mmol) Isobuten versetzt (Druckapparatur). Das Gemisch wird in dem verschlossenen Gefäß auf 20°C erwärmt und über 20 Stunden umgesetzt Darauf kühlt man wieder auf -30°C ab, öffnet die Apparatur und rührt das Reaktionsgemisch bei 20°C in 300 ml 3 M Natronlauge / 400 ml Ether ein. Die wäßrige Phase wird mit Ether nachextrahiert, die organische Lösung mit Natriumsulfat getrocknet und eingedampft.
Ausbeute: 23,3 g (84,9 mmol).

### Beispiel IV

trans-2-(4-Brommethylphenyl)-cyclohexan-1-carbonsäure-tert.butylester 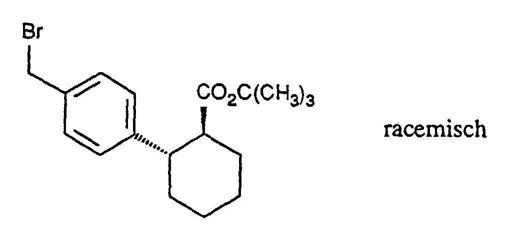 11,70 g (42,6 mmol) der Verbindung aus Beispiel III werden unter Rückfluß in 100 ml Tetrachlormethan mit 7,59 g (42,6 mmol) N-Bromsuccinimid und 1,4 g Azobisisobutyronitril umgesetzt. Nach einer Reaktionszeit von 4 h wird die Mischung abgekühlt, der angefallene Succinimidniederschlag abgesaugt und das Filtrat eingedampft.
Ausbeute: 14,2 g (40,2 mmol)
R_{f} = 0,48 (Petrolether : Essigester = 10:1)

### Beispiel V

4,6-Dimethyl-2-hydrazino-pyridin 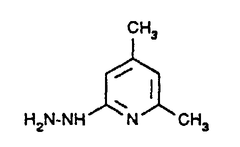 37,0 g (260 mmol) 2-Chlor-4,6-dimethyl-pyridin (US 36 32 807) werden 15 Stunden unter Rückfluß mit 67 ml Hydrazinhydrat in 200 ml Diethylenglykol gekocht. Nach dem Abkühlen auf Raumtemperatur extrahiert man mit Wasser und Ether/Dichlormethan, trocknet die organischen Phasen mit Natriumsulfat und dampft ein. Das rohe Produkt kann direkt weiter umgesetzt werden.
R_{f} = 0,18 (Dichlormethan: Methanol = 10:1)

### Beispiel VI

2,4-Dimethyl-5,6,7,8-tetrahydro-α-carbolin 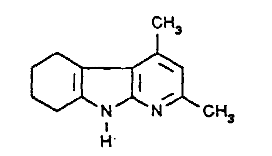 1,08 g (7,7 mmol) der Verbindung aus Beispiel V werden bei 20°C mit 0,86 ml (8,3 mmol) Cyclohexanon verrührt, und das nach einer Stunde erhaltene rohe Hydrazon ohne Reinigung weiter umgesetzt, indem es 20 Stunden in 30 ml Diethlyenglycol unter Rückfluß gekocht wird. Beim Abkühlen auf -10°C fällt ein Niederschlag aus, der abgesaugt und mit wenig gekühltem Methanol gewaschen wird. Nach Trocknen der Substanz im Hochvakuum über Phosphorpentoxid erhält man 0,32 g (1,6 mmol) Produkt.
R_{f} = 0,41 (Petrolether : Essigester = 1:1)
In Analogie zur Vorschrift des Beispiels VI werden die in Tabelle I aufgeführten Verbindungen hergestellt:

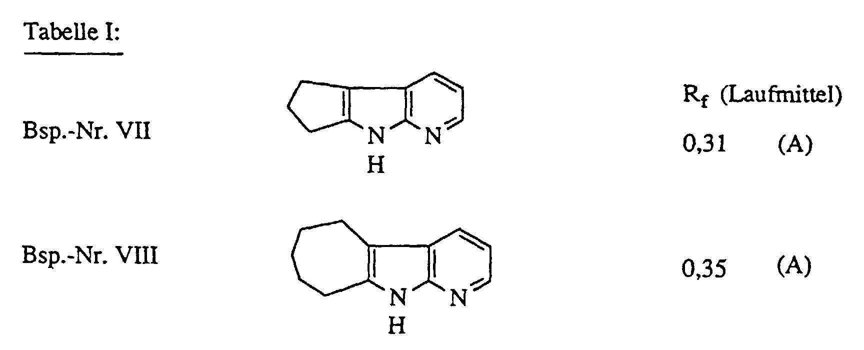

### Beispiel IX

trans-2-{4-[2,4-Dimethyl-5,6,7,8-tetrahydro-α-carbolin-9-yl-methyl]phenyl}-cyclohexan-1-carbonsäure-tert.butylester 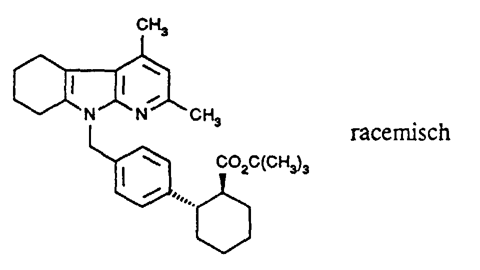 1,98 g (9,9 mmol) der Verbindung aus Beispiel VI werden in 70 ml wasserfreiem Dimethylformamid mit 298 mg (9,9 mmol) Natriumhydrid (80%ig, stabilisiert mit Paraffin) umgesetzt (0°C) und nach beendeter Wasserstoffentwicklung 20 Stunden bei 25°C mit 3,5 g (9,9 mmol) der Verbindung aus Beispiel IV gerührt. Nach Wasserzugabe extrahiert man mehrfach mit Ether, trocknet die vereinigten organischen Phasen mit Natriumsulfat und chroamatographiert den beim Eindampfen erhaltenen Rückstand (Kieselgel 60, Merck, Petrolether : Essigester von 20:1 bis 10:1).
Ausbeute: 2,31 g (4,9 mmol).
R_{f} = 0,49 (Petrolether : Essigester = 10:1)

In Analogie zur Vorschrift des Beispiels IX werden die in Tabelle 1 aufgeführten Verbindungen hergestellt:

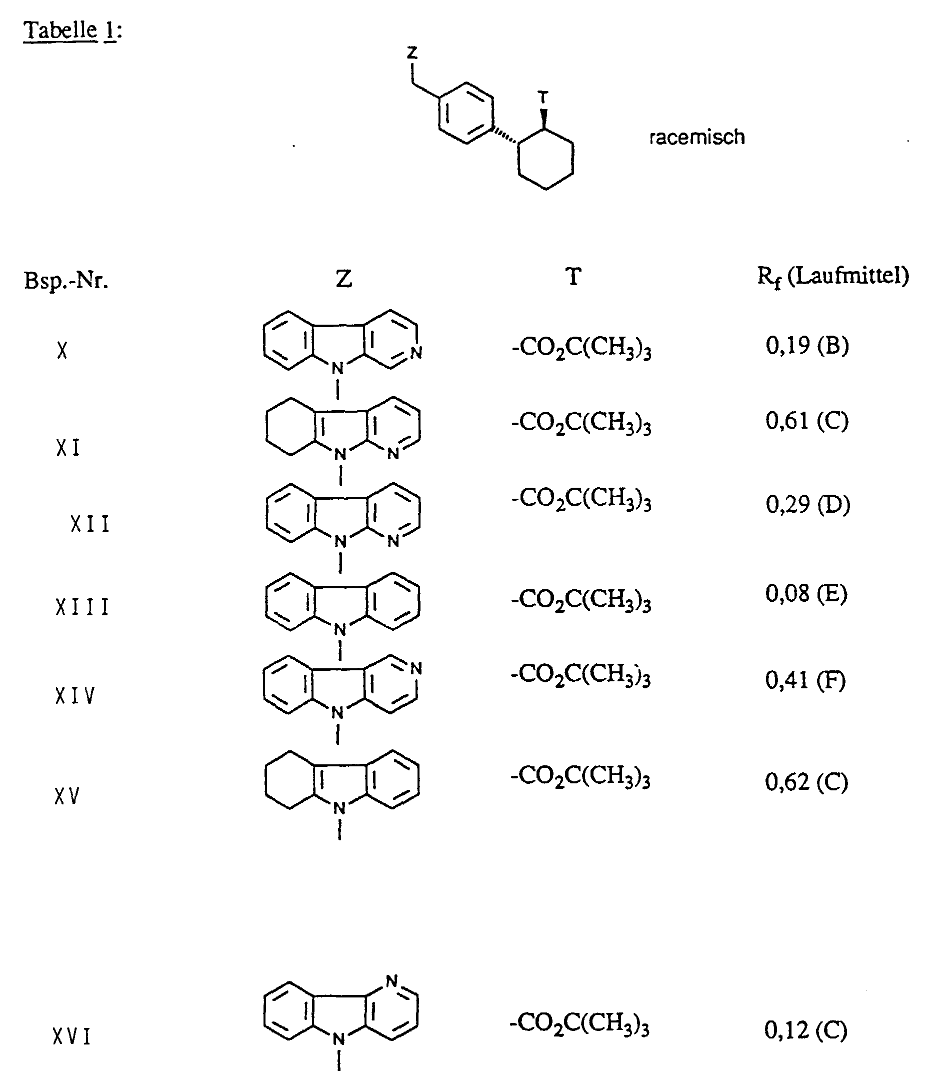

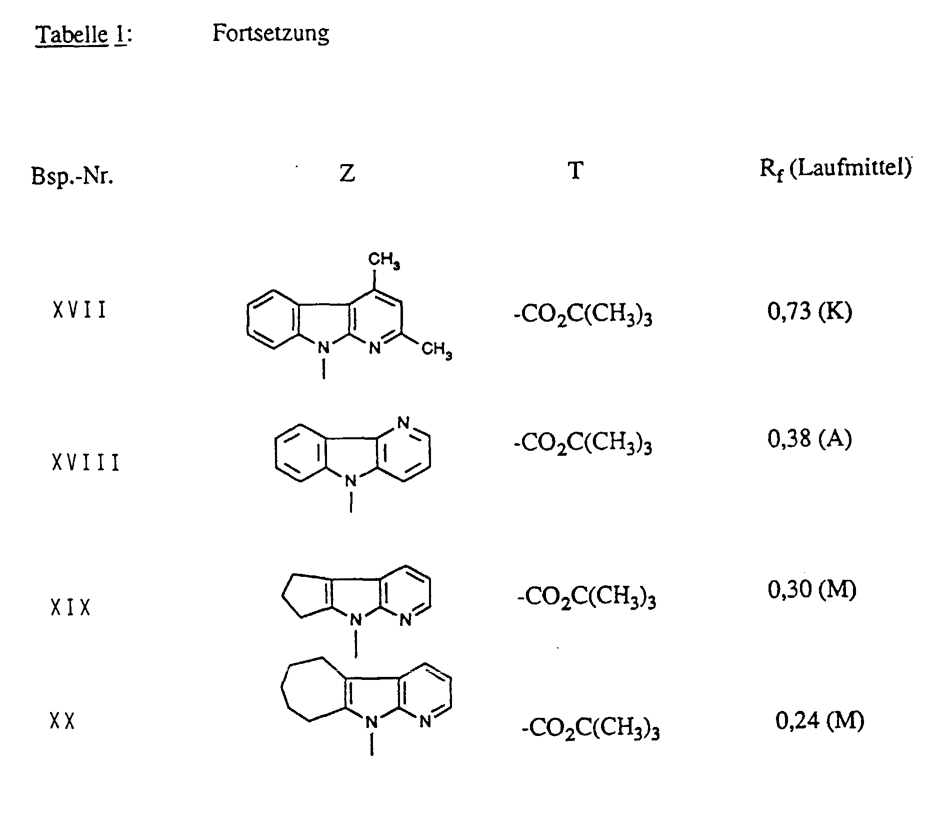

### Beispiel XXI

trans-2-{4-[2,4-Dimethyl-5,6,7,8-tetrahydro-α-carbolin-9-yl-methyl]phenyl}-cyclohexan-1-carbonsäure 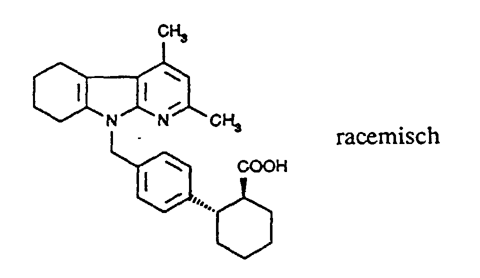 1,50 g (3,2 mmol) der Verbindung aus Beispiel XXI werden bei 20°C in 10 ml Dioxan mit 3 ml 37%iger Salzsäure zur Reaktion gebracht. Nach 3 Stunden setzt man Ether und Wasser zu und stellt mit Natriumcarbonatlösung auf pH = 8. Der anfallende Niederschlag wird abgesaugt, mit Wasser und Ether gewaschen und nach Trocknung im Hochvakuum über Phosphorpentoxid chromatographisch an Kieselgel 60 (Merck, Dichlormethan: Methanol = 50:1) gereinigt.
Ausbeute: 0,44 g (1,1 mmol)
R_{f}=0,63 (Dichlormethan: Methanol = 10.1)

In Analogie zur Vorschrift des Beispiels XXI werden die in Tabelle 2 aufgeführten Verbindungen hergestellt:

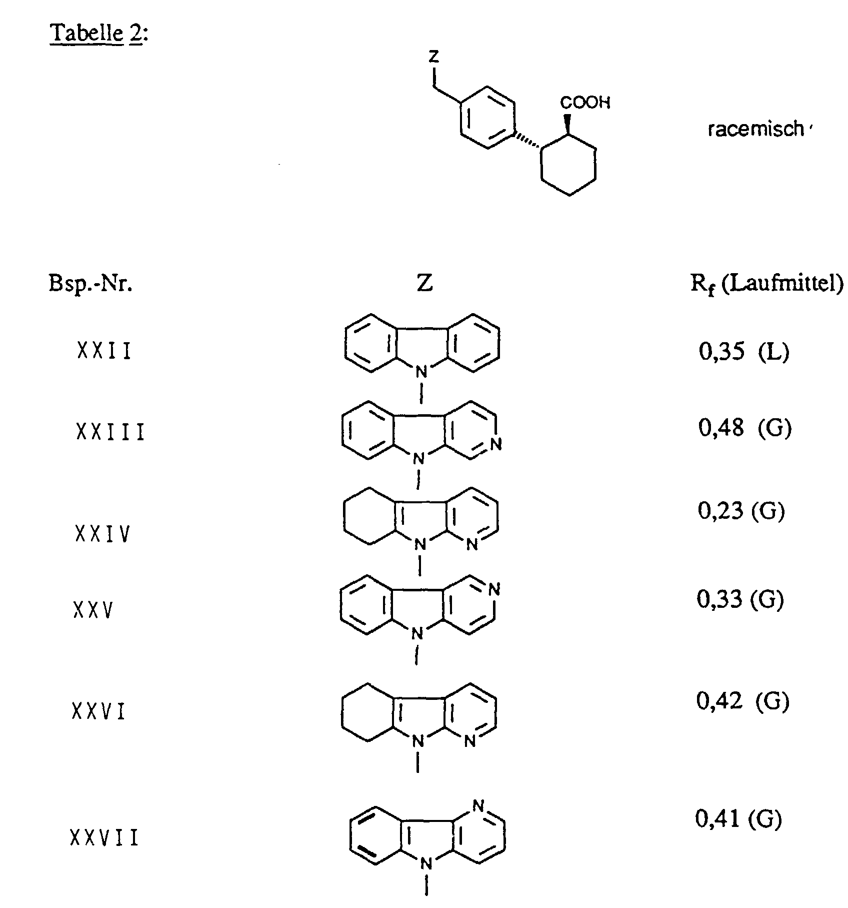

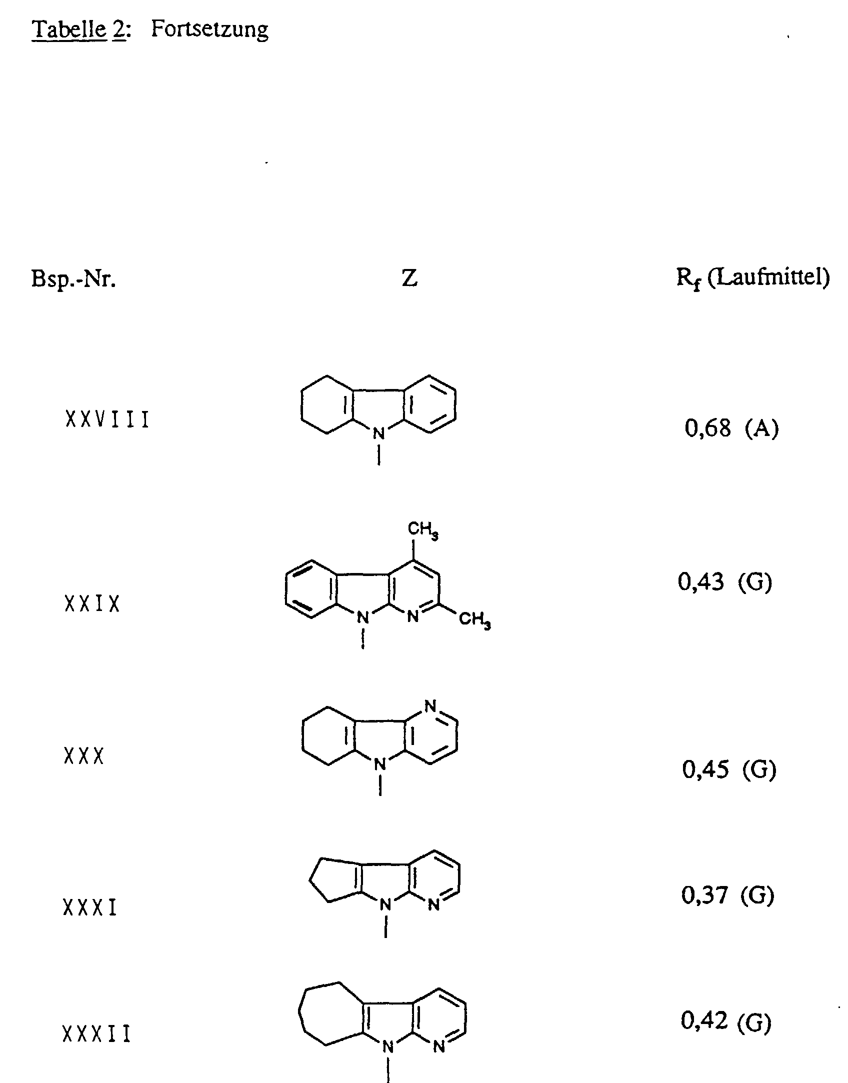

### Herstellungsbeispiele 1 und 2

trans-2-{4-[2,4-Dimethyl-5,6,7,8-tetrahydro-α-carbolin-9-yl-methyl]phenyl}-cyclohexan-1-carbonsäure-N((S)-phenylglycinol)amid 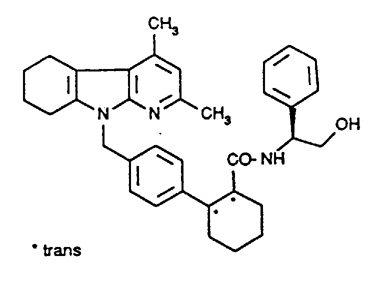 427 mg (1,03 mmol) der Verbindung aus Beispiel XXI werden bei - 30°C in 20 ml wasserfreiem Tetrahydrofuran mit 288 µl (2,10 mmol) Triethylamin und 87 µl (1,13 mmol) Mesylchlorid umgesetzt. Nach 30 Minuten gibt man 169 mg (1,23 mmol) (S)-Phenylglycinol und 126 mg (1,03 mmol) 4-(N,N-Dimethylamino)pyridin in 15 ml wasserfreiem Tetrahydrofuran zu und rührt unter Erwärmung auf 20°C 20 Stunden nach. Man setzt Wasser zu und extrahiert mehrfach mit Ether. Die organische Phase wird mit Natriumsulfat getrocknet und eingedampft; darauf wird das Rohprodukt an Kieselgel 60 (Merck, Petrolether : Essigester 2:1 bis 1:1) gereinigt
Ausbeuten:
110 mg (0,21 mmol) Diastereomer A (Beispiel 27); R_{f}= 0,29 (A)
40 mg (0,07 mmol) Diasteromer B (Beispiel 28); R_{f}= 0,15 (A)

In Analogie zu den Vorschriften der Beispiele 1 und 2 werden die in Tabelle 3 aufgeführten Verbindungen hergestellt:

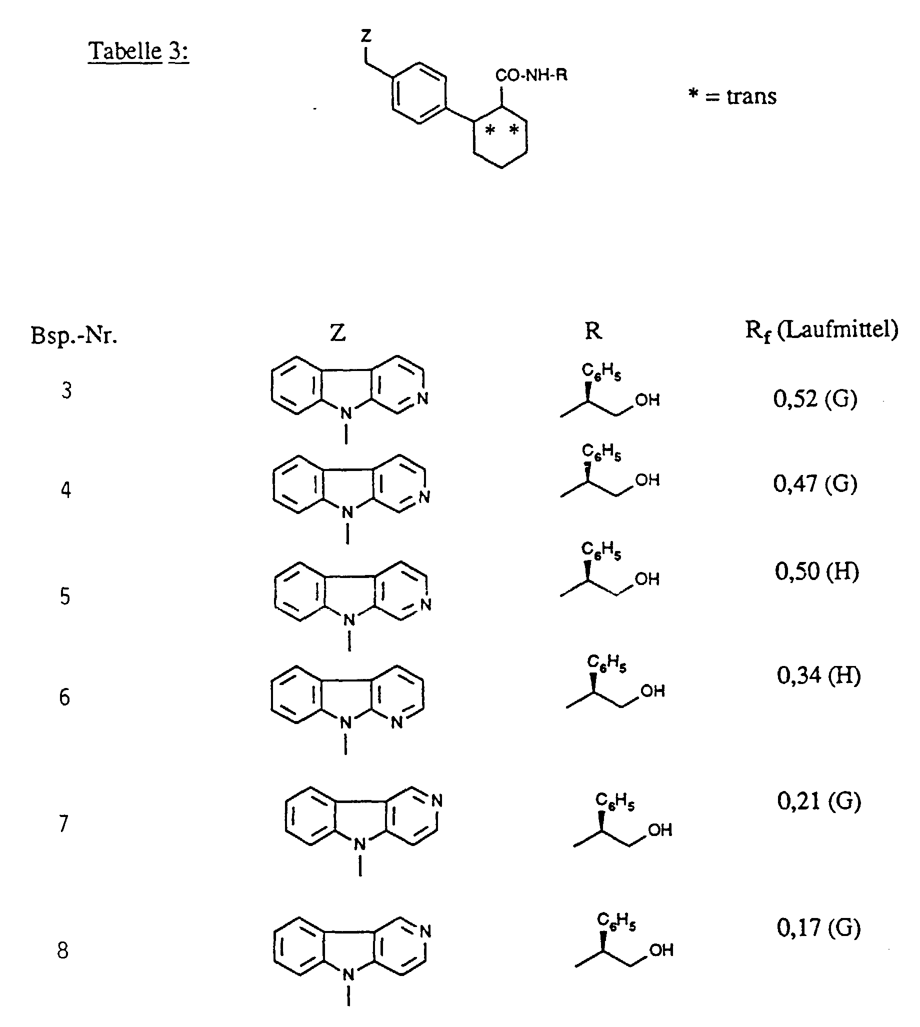

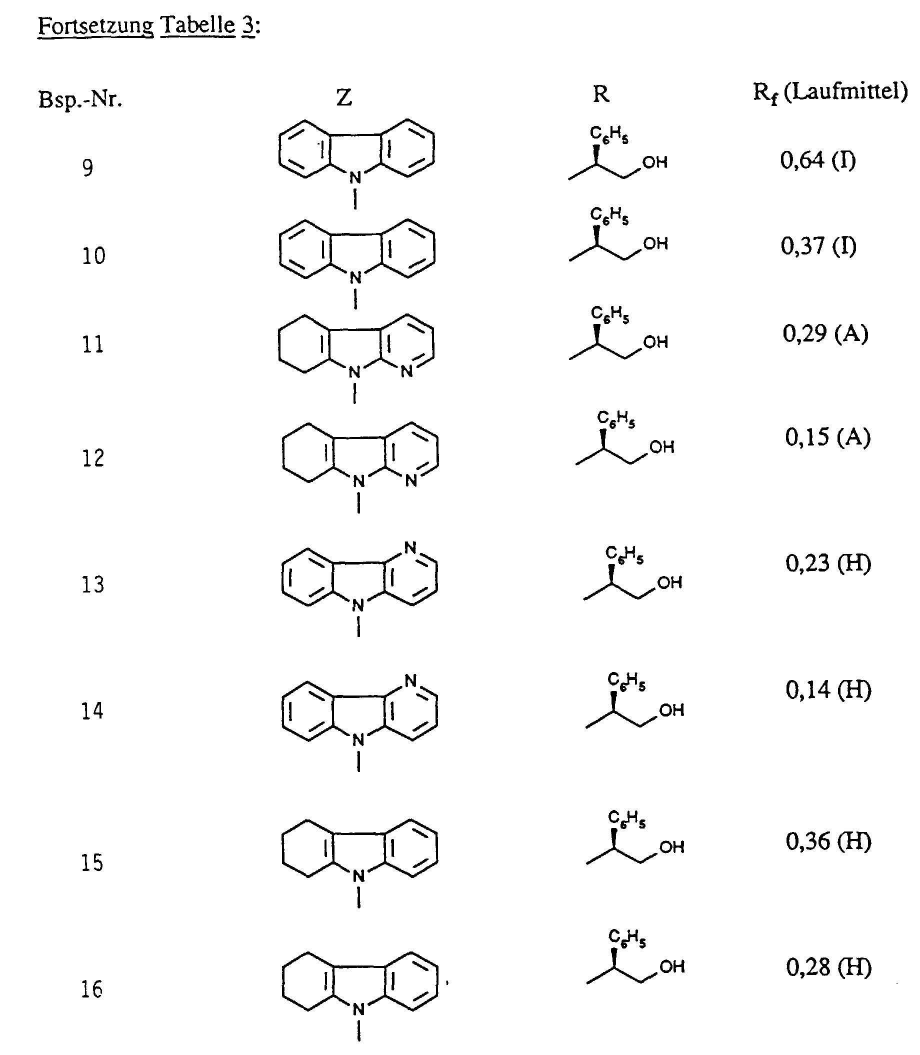

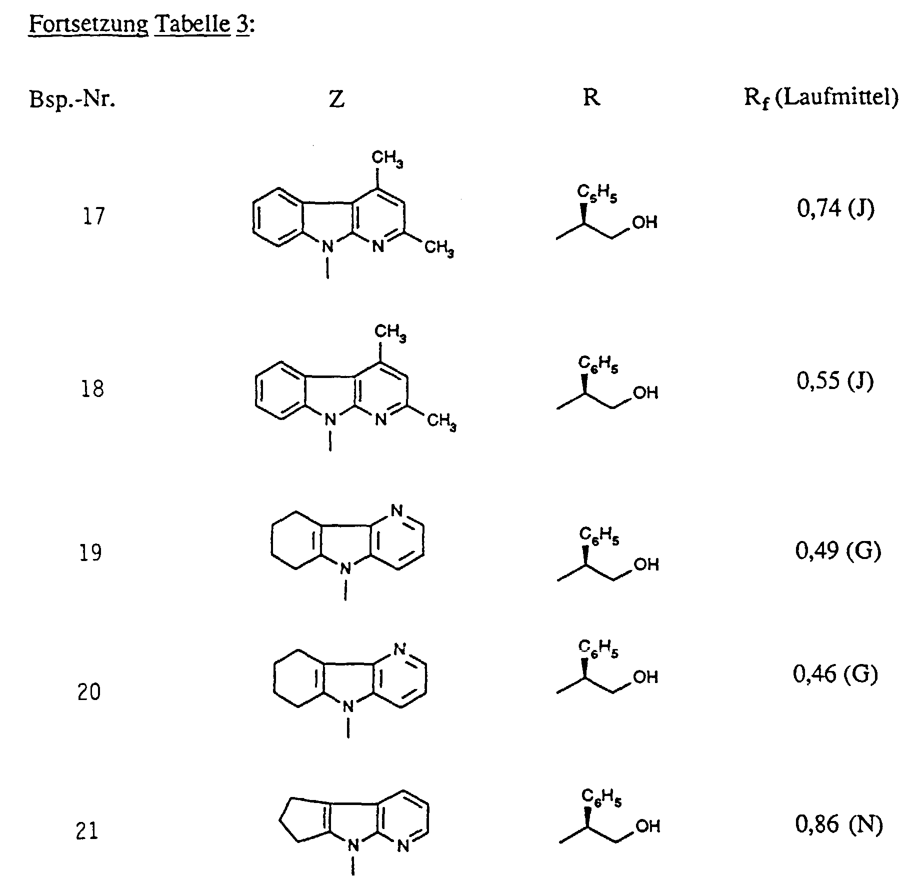

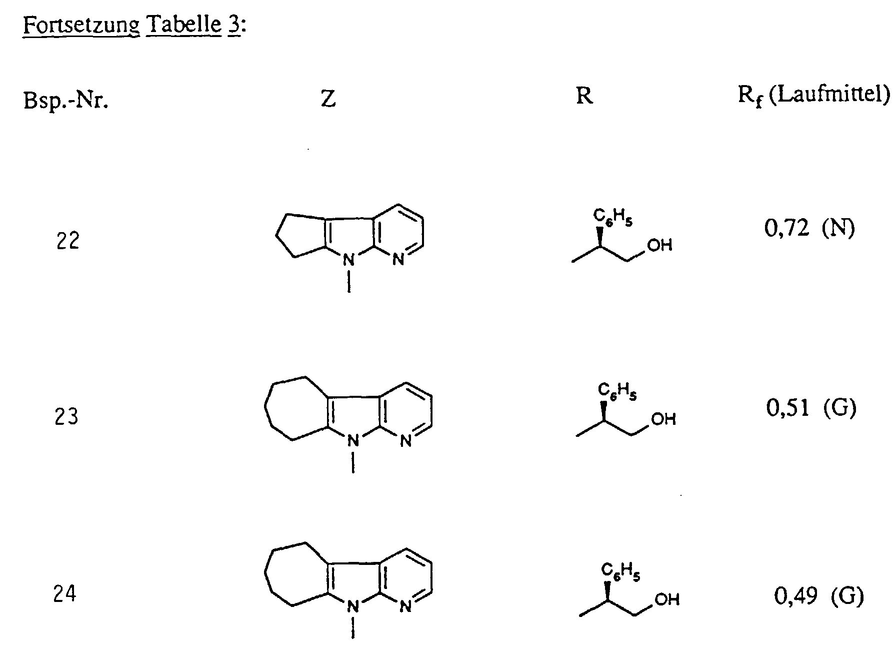

## Patentansprüche

1. Hetero-tricyclisch-substituierte Phenyl-cyclohexan-carbonsäurederivate der allgemeinen Formel 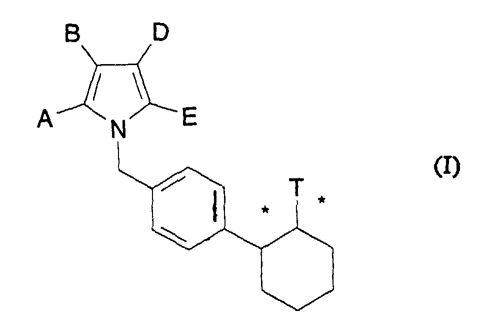 in welcher
A und B gemeinsam einen Rest der Formel 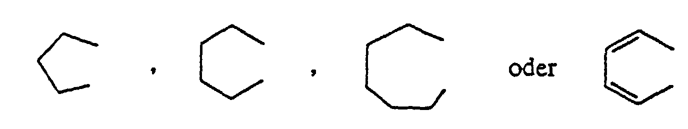 bilden,
D und E gemeinsam einen Rest der Formel 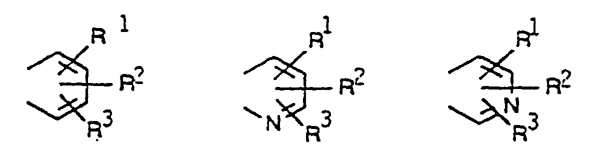 bilden, 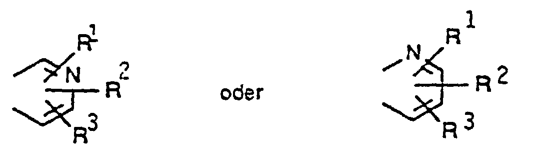 worin
R¹, R² und R³ gleich oder verschieden sind und Wasserstoff oder für Aryl mit 6 bis 10 Kohlenstoffatomen bedeuten, das gegebenenfalls durch Hydroxy, Halogen, Trifluormethyl oder durch geradkettiges oder verzweigtes Alkyl oder Alkoxy mit jeweils bis zu 6 Kohlenstoffatomen substiutiert ist, oder geradkettiges oder verzweigtes Alkyl oder Alkenyl mit jeweils bis zu 8 Kohlenstoffatomen bedeuten, oder Cycloalkyl mit 3 bis 8 Kohlenstoffatomen bedeuten, oder Halogen bedeuten,
T für einen Rest der Formel 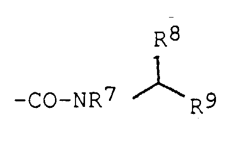 steht,
worin
R⁷ Wasserstoff oder geradkettiges oder verzweigtes Alkyl mit bis zu 6 Kohlenstoffatomen beteutet,
R⁸ Aryl mit 6 bis 10 Kohlenstoffatomen bedeutet, das gegebenenfalls bis zu 2-fach gleich oder verschieden durch Halogen, Hydroxy, geradkettiges oder verzweigtes Alkyl, Alkoxy oder Alkoxycarbonyl mit jeweils bis zu 6 Kohlenstoffatomen, Carboxy, Phenoxy oder C₃-C₆-Cycloalkyloxy substituiert ist,
R⁹ eine Gruppe der Formel -CH₂-OR¹⁰, -CO₂R¹¹, -CO-NR¹²R¹³ oder Pyridyl bedeutet,
worin
R¹⁰ Wasserstoff oder geradkettiges oder verzweigtes Alkyl mit bis zu 8 Kohlenstoffatomen bedeutet,
R¹¹ Wasserstoff, geradketiiges oder verzweigtes Alkyl mit bis zu 8 Kohlenstoffatomen, Phenyl oder Cycloalkyl mit 3 bis 6 Kohlenstoffatomen bedeutet,
R¹² und R¹³ gleich oder verschieden sind und Wasserstoff, geradkettiges oder verzweigtes Alkyl mit bis zu 8 Kohlenstoffatomen oder Phenyl bedeuten,
gegebenenfalls in einer isomeren Form, und deren Salze.

2. Hetero-tricyclisch-substituierte Phenyl-cyclohexan-carbonsäurederivate nach Anspruch 1
wobei
A und B gemeinsam einen Rest der Formel 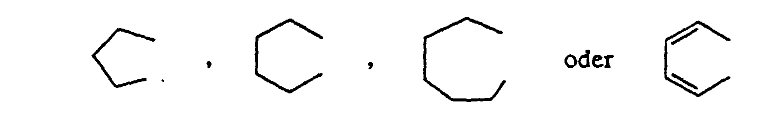 bilden,
D und E gemeinsam einen Rest der Formel 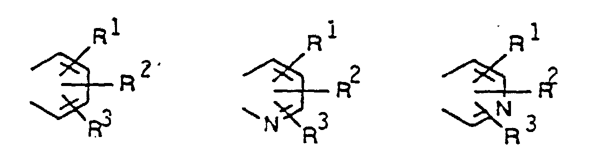 bilden, 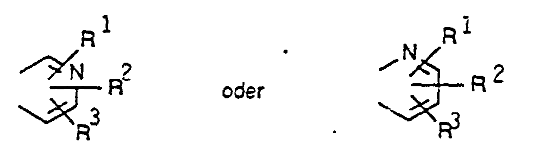 worin
R¹, R² und R³ gleich oder verschieden sind und Wasserstoff oder für Phenyl bedeuten, das gegebenenfalls durch Hydroxy, Fluor, Chlor, Brom, Trifluormethyl oder durch geradkettiges oder verzweigtes Alkyl oder Alkoxy mit jeweils bis zu 4 Kohlenstoffatomen substituiert ist, oder
geradkettiges oder verzweigtes Alkyl oder Alkenyl mit jeweils bis zu 6 Kohlenstoffatomen bedeuten, oder
Cyclopropyl, Cyclobutyl, Cyclopentyl, Cyclohexyl oder Cycloheptyl bedeuten, oder
Fluor, Chlor oder Brom bedeuten,
T für einen Rest der Formel 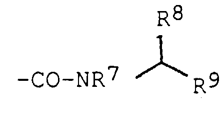 steht,
worin
R⁷ Wasserstoff oder geradkettiges oder verzweigtes Alkyl mit bis zu 4 Kohlenstoffatomen bedeutet,
R⁸ Phenyl bedeutet, das gegebenenfalls durch Fluor, Chlor, Brom, Hydroxy, geradkettiges oder verzweigtes Alkyl, Alkoxy oder Alkoxycarbonyl mit jeweils bis zu 4 Kohlenstoffatomen, Carboxy oder Phenoxy substituiert ist,
R⁹ eine Gruppe der Formel -CH₂-OR¹⁰, -CO₂R¹¹, -CO-NR¹²R¹³ oder Pyridyl bedeutet,
worin
R¹⁰ Wasserstoff oder geradkettiges oder verzweigtes Alkyl mit bis zu 6 Kohlenstoffatomen bedeutet,
R¹¹ Wasserstoff, geradkettiges oder verzweigtes Alkyl mit bis zu 6 Kohlenstoffatomen, Phenyl, Cyclopropyl, Cyclopentyl oder Cyclohexyl bedeutet,
R¹² und R¹³ gleich oder verschieden sind und Wasserstoff, geradkettiges oder verzweigtes Alkyl mit bis zu 6 Kohlenstoffatomen, Cyclopropyl, Cylcopentyl, Cylcohexyl oder Phenyl bedeuten,
gegebenenfalls in einer isomeren Form, und deren Salze.

3. Hetero-tricyclisch-substiyuierte Phenyl-cyclohexan-carbonsäurederivate nach Anspruch 1
wobei
A und B gemeinsam einen Rest der Formel 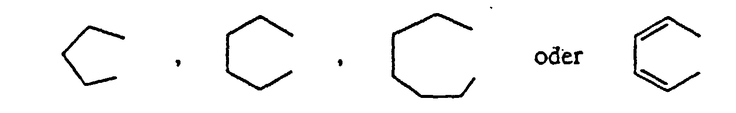 bilden,
D und E gemeinsam einen Rest der Formel 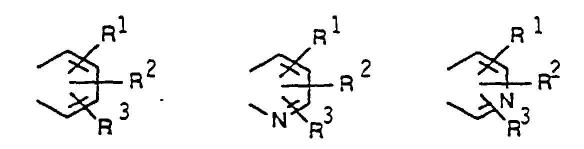 bilden, 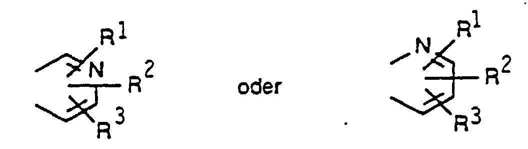 worin
R¹, R² und R³ gleich oder verschieden sind und Wasserstoff, Methyl, Fluor, Chlor oder Brom bedeuten,
T für einen Rest der Formel 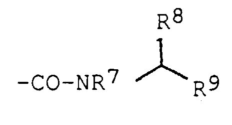 steht,
worin
R⁷ Wasserstoff oder Methyl bedeutet,
R⁸ Phenyl bedeutet, das gegebenenfalls durch Fluor, Chlor, Brom, Hydroxy, geradkettiges oder verzweigtes Alkyl, Alkoxy oder Alkoxycarbonyl mit jeweils bis zu 3 Kohlenstoffatomen, Carboxy oder Phenoxy substituiert ist,
R⁹ eine Gruppe der Formel -CH₂-OR¹⁰, -CO₂-R¹¹, -CO-NR¹²R¹³ oder Pyridyl bedeutet,
worin
R¹⁰ Wasserstoff oder geradkettiges oder verzweigtes Alkyl mit bis zu 4 Kohlenstoffatomen bedeutet,
R¹¹ Wasserstoff, geradkettiges oder verzweigtes Alkyl mit bis zu 4 Kohlenstoffatomen, Phenyl, Cyclopropyl, Cyclopentyl oder Cyclohexyl bedeutet,
R¹² und R¹³ gleich oder verschieden sind und Wasserstoff, geradkettiges oder verzweigtes Alkyl mit bis zu 4 Kohlenstoffatomen, Cyclopropyl, Cyclopentyl, Cyclohexyl oder Phenyl bedeuten,
gegebenenfalls in einer isomeren Form, und deren Salze.

4. Hetero-tricyclisch-substituierte Phenylcyclohexan-carbonsäurederivate nach Anspruch 1
worin
A und B gemeinsam einen Rest der Formel 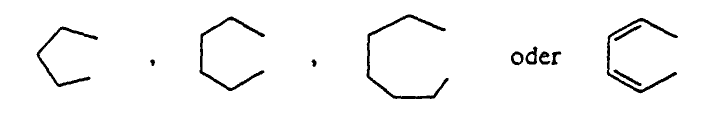 bilden,
D und E gemeinsam einen Rest der Formel 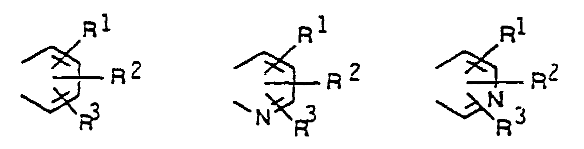 bilden, 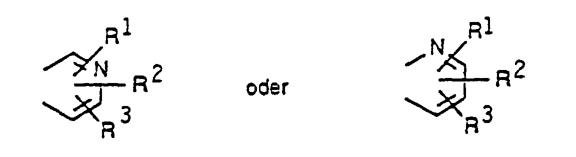 worin
R¹, R² und R³ gleich oder verschieden sind und für Wasserstoff, Brom oder Methyl stehen,
T für einen Rest der Formel 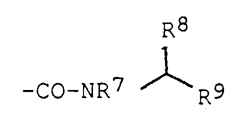 steht, worin
R⁷ Wasserstoff bedeutet,
R⁸ Phenyl bedeutet,
R⁹ eine Gruppe -CH₂OH bedeutet,
gegebenenfalls in einer isomeren Form, und deren Salze.

5. Hetero-tricyclisch-substituierte Phenyl-cyclohexan-carbonsäurederivate nach den Ansprüchen 1 bis 4 zur therapeutischen Anwendung.

6. Verfahren zur Herstellung von hetero-tricyclisch-substituierten Phenylcyclohexan-carbonsäurederivaten nach den Ansprüchen 1 bis 4, dadurch gekennzeichnet, daß man
Verbindungen der allgemeinen Formel (II) 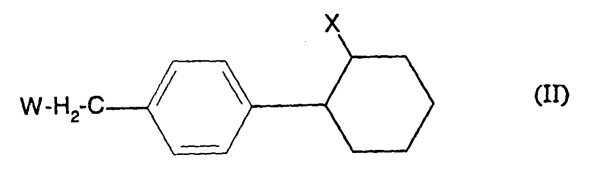 in welcher
W für eine typische Abgangsgruppe wie beispielsweise Chlor, Brom, Jod, Tosylat oder Mesylat, vorzugsweise für Brom steht
und
X für C₁-C₆-Alkoxycarbonyl steht,
zunächst mit Verbindungen der allgemeinen Formel (III) 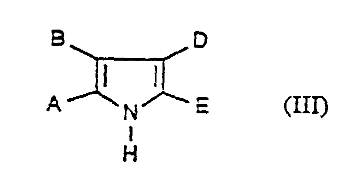 in welcher
A, B, D und E die oben angegebene Bedeutung haben,
in inerten Lösemitteln, gegebenenfalls in Anwesenheit einer Base und gegebenenfalls unter Schutzgasatmosphäre zu Verbindungen der allgemeinen Formel (IV) 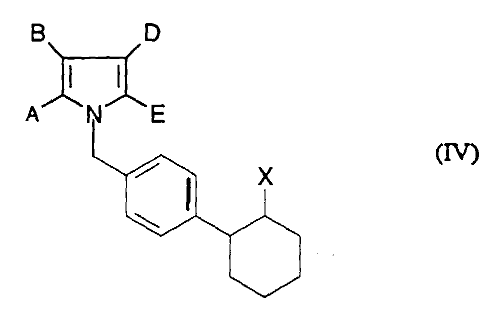 in welcher
A, B, D, E und X die oben angebebene Bedeutung haben,
umsetzt, und gegebenenfalls ausgehend von den entsprechenden Carbonsäuren, nach vorgeschalteter Hydrolyse und/oder Aktivierung, anschließend Aminen der allgemeinen Formeln (Va) 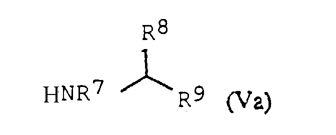 in welchen
R⁷, R⁸ und R⁹ die oben angegebene Bedeutung haben,
gegebenenfalls in Anwesenheit einer Base und/oder eines Hilfsmittels, beispielsweise eines Dehydratisierungsmittels, in inerten Lösemitteln amidiert,
und gegebenenfalls die Isomeren trennt, und im Fall der Herstellung der Salze mit einer entsprechenden Base oder Säure umsetzL

7. Arzneimittel enthaltend mindestens ein hetero-tricyclisch-substituiertes Phenyl-cyclohexan-carbonsäurederivat nach Anspruch 1 bis 4.

8. Arzneimittel nach Anspruch 7 zur Behandlung von arteriellem Bluthochdruck und Atherosklerose.

9. Verfahren zur Herstellung eines Arzneimittels nach Anspruch 7 dadurch gekennzeichnet, daß man die Wirkstoffe gegebenenfalls mit Hilfe von geeigneten Hilfs- und Trägerstoffen in eine geeignete Applikationsform überführt.

10. Verwendung von hetero-tricyclisch-substituierten Phenyl-cyclohexancarbonsäurederivaten nach Ansprüchen 1 bis 4 zur Herstellung von Arzneimitteln.

## Claims

1. Heterotricyclically substituted phenyl-cyclohexanecarboxylic acid derivatives of the general formula 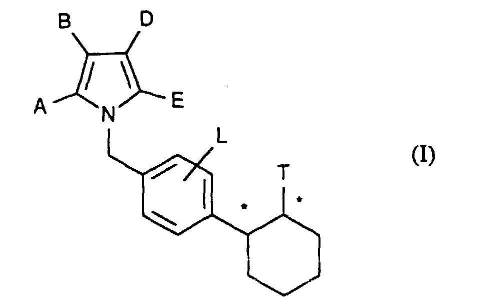 in which
A and B together form a radical of the formula 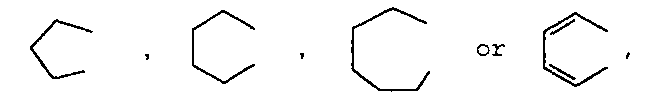
D and E together form a radical of the formula 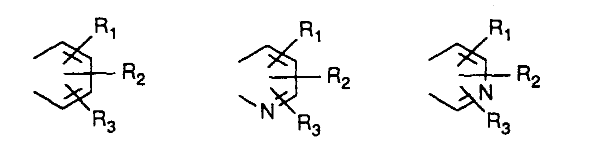 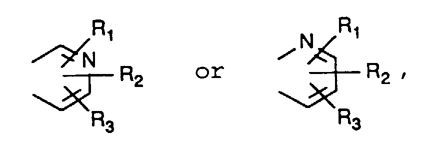
in which
R¹, R² and R³ are identical or different and denote hydrogen or aryl having 6 to 10 carbon atoms, which is optionally substituted by hydroxyl, halogen or trifluoromethyl or by straight-chain or branched alkyl or alkoxy in each case having up to 6 carbon atoms, or
denote straight-chain or branched alkyl or alkenyl in each case having up to 8 carbon atoms, or denote cycloalkyl having 3 to 8 carbon atoms, or denote halogen,
T represents a radical of the formula 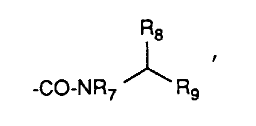
in which
R⁷ denotes hydrogen or straight-chain or branched alkyl having up to 6 carbon atoms,
R⁸ denotes aryl having 6 to 10 carbon atoms, which is optionally substituted up to 2 times by identical or different substituents from the group consisting of halogen, hydroxyl, straight-chain or branched alkyl, alkoxy or alkoxycarbonyl in each case having up to 6 carbon atoms, carboxyl, phenoxy and C₃-C₆-cycloalkoxy,
R⁹ denotes a group of the formula -CH₂-OR¹⁰, -CO₂R¹¹, -CO-NR¹²R¹³ or pyridyl,
in which
R¹⁰ denotes hydrogen or straight-chain or branched alkyl having up to 8 carbon atoms,
R¹¹ denotes hydrogen, straight-chain or branched alkyl having up to 8 carbon atoms, or phenyl or cycloalkyl having 3 to 6 carbon atoms,
R¹² and R¹³ are identical or different and denote hydrogen, straight-chain or branched alkyl having up to 8 carbon atoms, or phenyl,
if appropriate in an isomeric form, and their salts.

2. Heterotricyclically substituted phenyl-cyclohexanecarboxylic acid derivatives according to Claim 1
in which
A and B together form a radical of the formula 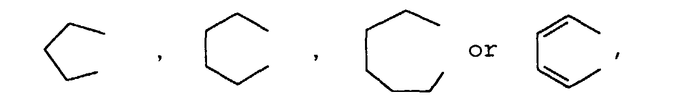
D and E together form a radical of the formula 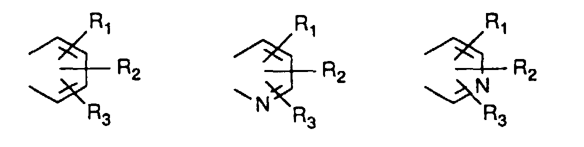 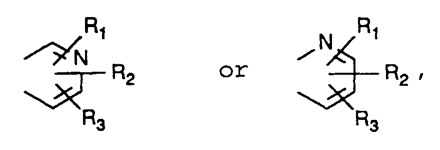
in which
R¹, R² and R³ are identical or different and denote hydrogen or phenyl which is optionally substituted by hydroxyl, fluorine, chlorine, bromine or trifluoromethyl or by straight-chain or branched alkyl or alkoxy in each case having up to 4 carbon atoms, or
denote straight-chain or branched alkyl or alkenyl in each case having up to 6 carbon atoms, or
denote cyclopropyl, cyclobutyl, cyclopentyl, cyclohexyl or cycloheptyl, or
denote fluorine, chlorine or bromine,
T represents a radical of the formula 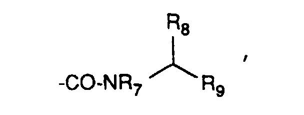
in which
R⁷ denotes hydrogen or straight-chain or branched alkyl having up to 4 carbon atoms,
R⁸ denotes phenyl which is optionally substituted by fluorine, chlorine, bromine, hydroxyl, straight-chain or branched alkyl, alkoxy or alkoxycarbonyl in each case having up to 4 carbon atoms, carboxyl or phenoxy,
R⁹ denotes a group of the formula -CH₂-OR¹⁰, -CO₂R¹¹, -CO-NR¹²R¹³ or pyridyl,
in which
R¹⁰ denotes hydrogen or straight-chain or branched alkyl having up to 6 carbon atoms,
R¹¹ denotes hydrogen, straight-chain or branched alkyl having up to 6 carbon atoms, phenyl, cyclopropyl, cyclopentyl or cyclohexyl,
R¹² and R¹³ are identical or different and denote hydrogen, straight-chain or branched alkyl having up to 6 carbon atoms, cyclopropyl, cyclopentyl, cyclohexyl or phenyl,
if appropriate in an isomeric form, and their salts.

3. Heterotricyclically substituted phenyl-cyclohexane-carboxylic acid derivatives according to Claim 1
in which
A and B together form a radical of the formula 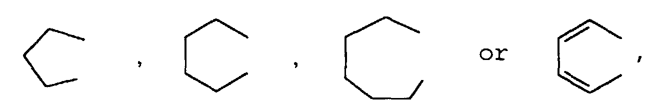
D and E together form a radical of the formula 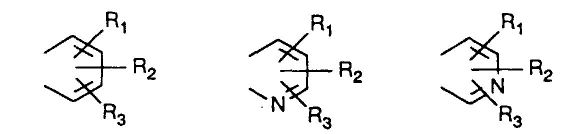 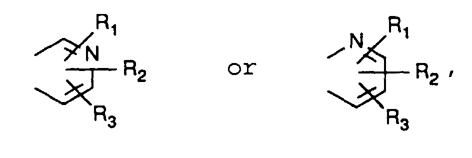 in which
R¹, R² and R³ are identical or different and denote hydrogen, methyl, fluorine, chlorine or bromine,
T represents a radical of the formula 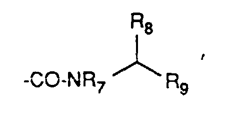 in which
R⁷ denotes hydrogen or methyl,
R⁸ denotes phenyl which is optionally substituted by fluorine, chlorine, bromine, hydroxyl, straight-chain or branched alkyl, alkoxy or alkoxycarbonyl in each case having up to 3 carbon atoms, carboxyl or phenoxy,
R⁹ denotes a group of the formula -CH₂-OR¹⁰, -CO₂-R¹¹, -CO-NR¹²R¹³ or pyridyl,
in which
R¹⁰ denotes hydrogen or straight-chain or branched alkyl having up to 4 carbon atoms,
R¹¹ denotes hydrogen or straight-chain or branched alkyl having up to 4 carbon atoms, phenyl, cyclopropyl, cyclopentyl or cyclohexyl,
R¹² and R¹³ are identical or different and denote hydrogen or straight-chain or branched alkyl having up to 4 carbon atoms, cyclopropyl, cyclopentyl, cyclohexyl or phenyl,
if appropriate in an isomeric form, and their salts.

4. Heterocyclically substituted phenyl-cyclohexanecarboxylic acid derivatives according to Claim 1
in which
A and B together form a radical of the formula 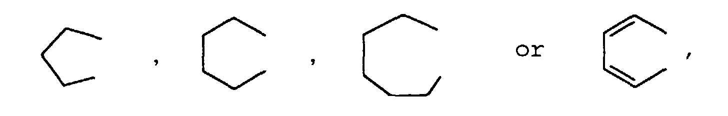
D and E together form a radical of the formula 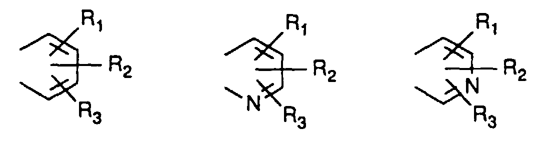 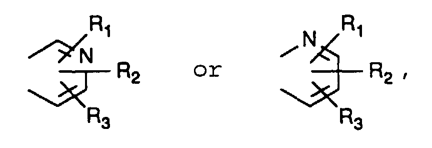 in which
R¹, R² and R³ are identical or different and denote hydrogen, bromine or methyl,
T represents a radical of the formula 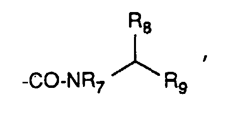
in which
R⁷ denotes hydrogen,
R⁸ denotes phenyl,
and
R⁹ denotes a group -CH₂OH,
if appropriate in an isomeric form, and their salts.

5. Heterotricyclically substituted phenyl-cyclohexane-carboxylic acid derivatives according to Claims 1 to 4 for therapeutic use.

6. Process for the preparation of heterotricyclically substituted phenyl-cyclohexane-carboxylic acid derivatives according to Claims 1 to 4, characterized in that compounds of the general formula (II) 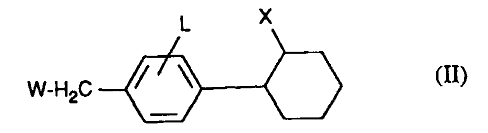 in which
W represents a typical leaving group such as, for example, chlorine, bromine, iodine, tosylate or mesylate, preferably bromine
and
X represents C₁-C₆-alkoxycarbonyl,
are reacted first with compounds of the general formula (III) 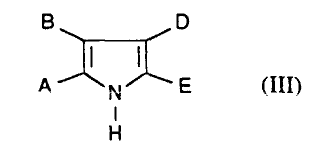 in which
A, B, D and E have the abovementioned meaning,
in inert solvents, if appropriate in the presence of a base and if appropriate under a protective gas atmosphere, to give compounds of the general formula (IV) 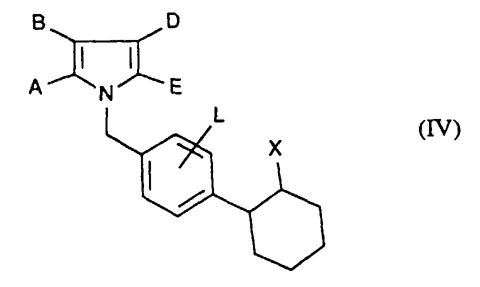 in which
A, B, D, E and X have the abovementioned meaning,
and if appropriate starting from the corresponding carboxylic acids, the products are subsequently amidated in inert solvents, after prior hydrolysis and/or activation, with amines of the general formula (Va) 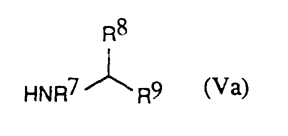 in which
R⁷, R⁸ and R⁹ have the abovementioned meaning,
if appropriate in the presence of a base and/or of an auxiliary, for example of a dehydrating agent,
and if appropriate the isomers are separated, and in the case of the preparation of the salts reacted with an appropriate base or acid.

7. Medicaments containing at least one heterotricyclically substituted phenyl-cyclohexane-carboxylic acid derivative according to Claims 1 to 4.

8. Medicaments according to Claim 7 for the treatment of arterial hypertension and atherosclerosis.

9. Process for the production of a medicament according to Claim 7, characterized in that the active compounds are converted into a suitable administration form, if appropriate with the aid of suitable auxiliaries and excipients.

10. Use of heterotricyclically substituted phenyl-cyclohexane-carboxylic acid derivatives according to Claims 1 to 4 for the production of medicaments.

## Revendications

1. Dérivés d'acides phényl-cyclohexane-carboxyliques substitués par un ou plusieurs composés hétéro-tricycliques, répondant à la formule générale 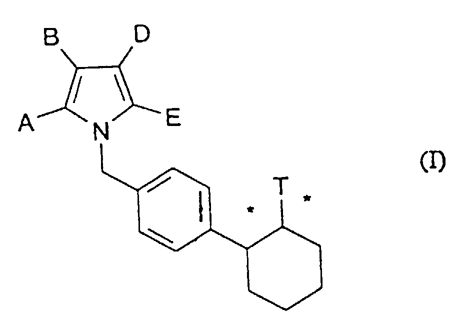 dans laquelle
A et B forment ensemble un radical répondant aux formules 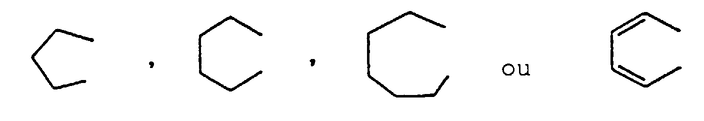
D et E forment ensemble un radical répondant aux formules 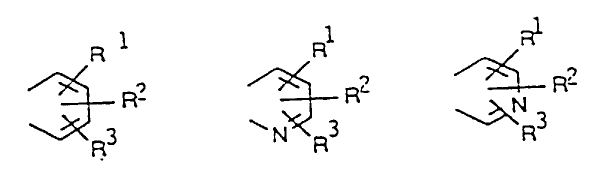 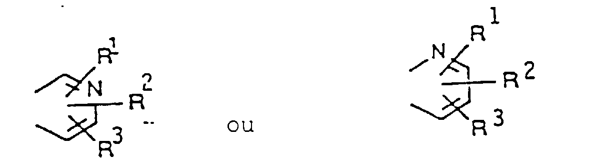
dans lesquelles
R¹, R² et R³ sont identiques ou différents et représentent un atome d'hydrogène ou un groupe aryle contenant de 6 à 10 atomes de carbone, qui porte éventuellement un ou plusieurs substituants identiques ou différents hydroxyle, halogéno, trifluorométhyle ou encore alkyle ou alcoxy à chaîne droite ou ramifiée contenant respectivement jusqu'à 6 atomes de carbone, ou représentent un groupe alkyle ou un groupe alcényle à chaîne droite ou ramifiée contenant respectivement jusqu'à 8 atomes de carbone, ou représentent un groupe cycloalkyle contenant de 3 à 8 atomes de carbone ou encore un atome d'halogène,
T représente un radical de formule 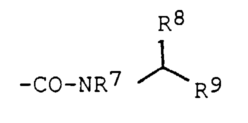 dans laquelle
R⁷ représente un atome d'hydrogène ou un groupe alkyle à chaîne droite ou ramifiée contenant jusqu'à 6 atomes de carbone,
R⁸ représente un groupe aryle contenant de 6 à 10 atomes de carbone, qui porte éventuellement jusqu'à deux substituants identiques ou différents halogéno; hydroxyle; alkyle, alcoxy ou alcoxycarbonyle à chaîne droite ou ramifiée, chacun de ces groupes contenant jusqu'à 6 atomes de carbone; carboxyle, phénoxy ou cycloalkyl(en C₃-C₆)oxy,
R⁹ représente un groupe de formules comprenant -CH₂-OR¹⁰, -CO₂R¹¹, -CO-NR¹²R¹³ ou encore un groupe pyridyle,
où
R¹⁰ représente un atome d'hydrogène ou encore un groupe alkyle à chaîne droite ou ramifiée contenant jusqu'à 8 atomes de carbone,
R¹¹ représente un atome d'hydrogène, un groupe alkyle à chaîne droite ou ramifiée contenant jusqu'à 8 atomes de carbone, un groupe phényle ou encore un groupe cycloalkyle contenant de 3 à 6 atomes de carbone,
R¹² et R¹³ sont identiques ou différents et représentent un atome d'hydrogène, un groupe alkyle à chaîne droite ou ramifiée contenant jusqu'à 8 atomes de carbone ou encore un groupe phényle,
éventuellement sous forme isomère, ainsi que leurs sels.

2. Dérivés d'acides phényl-cyclohexane-carboxyliques substitués par un ou plusieurs composés hétéro-tricycliques selon la revendication 1
dans lesquels
A et B forment ensemble un radical répondant aux formules 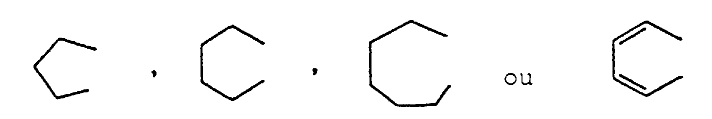
D et E forment ensemble un radical répondant aux formules 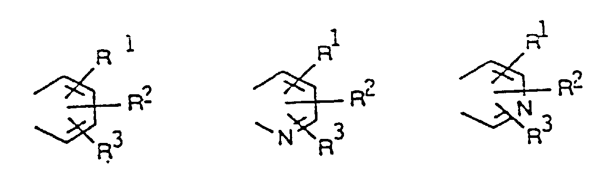 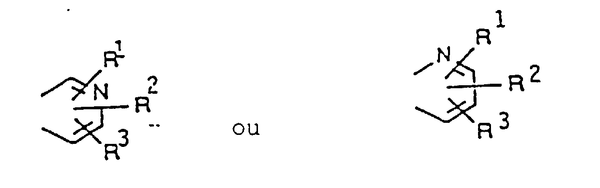
dans lesquelles
R¹, R² et R³ sont identiques ou différents et représentent un atome d'hydrogène ou un groupe phényle qui porte éventuellement un ou plusieurs substituants identiques ou différents hydroxyle; fluoro, chloro, bromo; trifluorométhyle; ou encore alkyle ou alcoxy à chaîne droite ou ramifiée contenant respectivement jusqu'à 4 atomes de carbone; ou représentent un groupe alkyle ou un groupe alcényle à chaîne droite ou ramifiée contenant respectivement jusqu'à 6 atomes de carbone; ou représentent un groupe cyclopropyle, un groupe cyclobutyle, un groupe cyclopentyle, un groupe cyclohexyle ou un groupe cycloheptyle ou encore un atome de fluor, un atome de chlore ou un atome de brome,
T représente un radical de formule 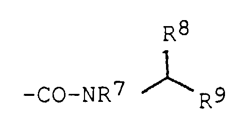 dans laquelle
R⁷ représente un atome d'hydrogène ou un groupe alkyle à chaîne droite ou ramifiée contenant jusqu'à 4 atomes de carbone,
R⁸ représente un groupe phényle qui porte éventuellement un ou plusieurs substituants identiques ou différents fluoro, chloro, bromo; hydroxyle; alkyle, alcoxy ou alcoxycarbonyle à chaîne droite ou ramifiée, chacun de ces groupes contenant jusqu'à 4 atomes de carbone; carboxyle ou phénoxy,
R⁹ représente un groupe de formules comprenant -CH₂-OR¹⁰, -CO₂R¹¹, -CO-NR¹²R¹³ ou encore un groupe pyridyle,
où
R¹⁰ représente un atome d'hydrogène ou encore un groupe alkyle à chaîne droite ou ramifiée contenant jusqu'à 6 atomes de carbone,
R¹¹ représente un atome d'hydrogène, un groupe alkyle à chaîne droite ou ramifiée contenant jusqu'à 6 atomes de carbone, un groupe phényle, un groupe cyclopropyle, un groupe cyclopentyle ou un groupe cyclohexyle,
R¹² et R¹³ sont identiques ou différents et représentent un atome d'hydrogène, un groupe alkyle à chaîne droite ou ramifiée contenant jusqu'à 6 atomes de carbone, un groupe cyclopropyle, un groupe cyclopentyle, un groupe cyclohexyle ou un groupe phényle,
éventuellement sous forme isomère, ainsi que leurs sels.

3. Dérivés d'acides phényl-cyclohexane-carboxyliques substitués par un ou plusieurs composés hétéro-tricycliques selon la revendication 1
dans lesquels
A et B forment ensemble un radical répondant aux formules 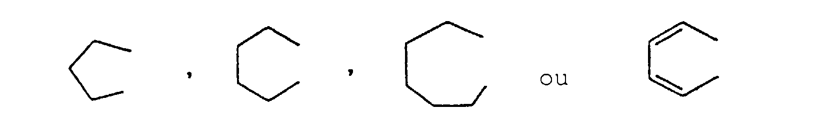
D et E forment ensemble un radical répondant aux formules 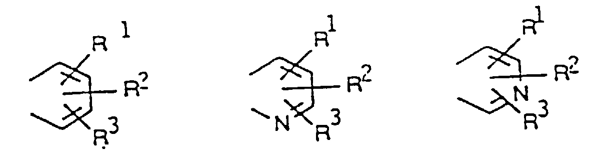 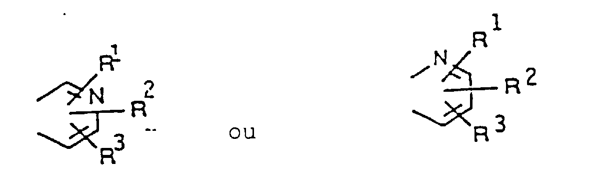
dans lesquelles
R¹, R² et R³ sont identiques ou différents et représentent un atome d'hydrogène, un groupe méthyle, un atome de fluor, un atome de chlore ou un atome de brome,
T représente un radical de formule 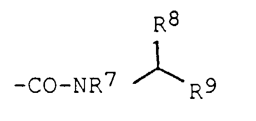 dans laquelle
R⁷ représente un atome d'hydrogène ou un groupe méthyle,
R⁸ représente un groupe phényle qui porte éventuellement un ou plusieurs substituants identiques ou différents fluoro, chloro, bromo; hydroxyle; alkyle, alcoxy ou alcoxycarbonyle à chaîne droite ou ramifiée, chacun de ces groupes contenant jusqu'à 3 atomes de carbone; carboxyle ou phénoxy,
R⁹ représente un groupe de formules comprenant -CH₂-OR¹⁰, -CO₂R¹¹, -CO-NR¹²R¹³ ou encore un groupe pyridyle,
où
R¹⁰ représente un atome d'hydrogène ou encore un groupe alkyle à chaîne droite ou ramifiée contenant jusqu'à 4 atomes de carbone,
R¹¹ représente un atome d'hydrogène, un groupe alkyle à chaîne droite ou ramifiée contenant jusqu'à 4 atomes de carbone, un groupe phényle, un groupe cyclopropyle, un groupe cyclopentyle ou un groupe cyclohexyle,
R¹² et R¹³ sont identiques ou différents et représentent un atome d'hydrogène, un groupe alkyle à chaîne droite ou ramifiée contenant jusqu'à 4 atomes de carbone, un groupe cyclopropyle, un groupe cyclopentyle, un groupe cyclohexyle ou un groupe phényle,
éventuellement sous forme isomère, ainsi que leurs sels.

4. Dérivés d'acides phényl-cyclohexane-carboxyliques substitués par un ou plusieurs composés hétéro-tricycliques selon la revendication 1
dans lesquels
A et B forment ensemble un radical répondant aux formules 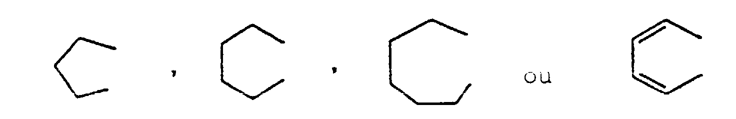
D et E forment ensemble un radical répondant aux formules 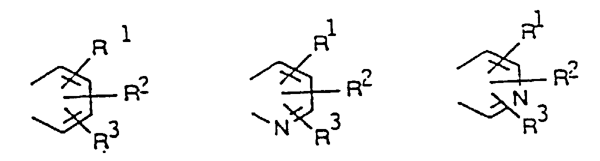 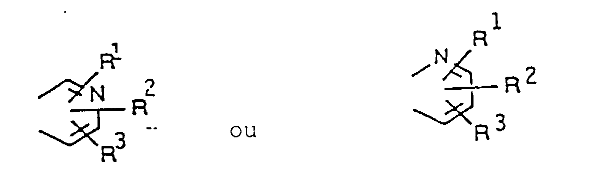
dans lesquelles
R¹, R² et R³ sont identiques ou différents et représentent un atome d'hydrogène, un atome de brome ou un groupe méthyle,
T représente un radical de formule 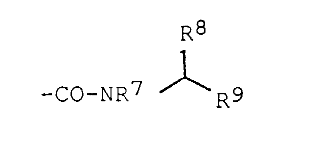 dans laquelle
R⁷ représente un atome d'hydrogène,
R⁸ représente un groupe phényle,
R⁹ représente un groupe -CH₂OH,
éventuellement sous forme isomère, ainsi que leurs sels.

5. Dérivés d'acides phényl-cyclohexane-carboxyliques substitués par un ou plusieurs composés hétéro-tricycliques selon les revendications 1 à 4 pour l'application thérapeutique.

6. Procédé pour la préparation de dérivés d'acides phényl-cyclohexane-carboxyliques substitués par un ou plusieurs composés hétéro-tricycliques selon les revendications 1 à 4, caractérisé en ce qu'on fait réagir des composés répondant à la formule générale (Il) 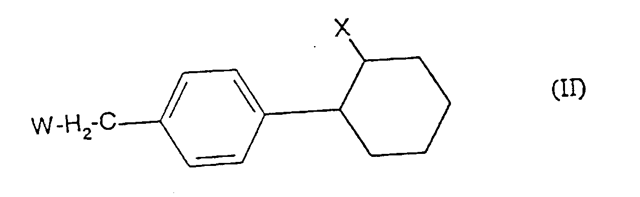 dans laquelle
W représente un groupe spécifique qui se sépare, tel que par exemple un atome de chlore, un atome de brome, un atome d'iode, un groupe tosylate ou un groupe mésylate, de préférence un atome de brome
et
X représente un groupe alcoxy(en C₁-C₆)carbonyle,
d'abord avec des composés répondant à la formule générale (III) 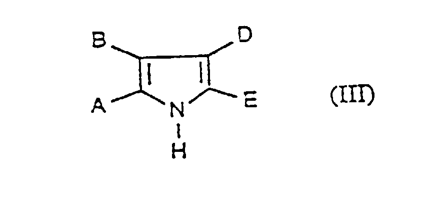 dans laquelle
A, B, D et E ont la signification indiquée ci-dessus,
dans des solvants inertes, éventuellement en présence d'une base et éventuellement sous l'atmosphère d'un gaz protecteur pour obtenir des composés répondant à la formule générale (IV) 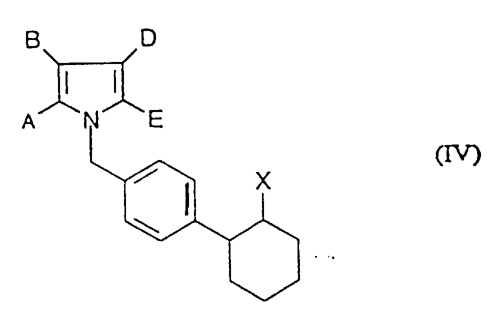 dans laquelle
A, B, D, E et X ont la signification indiquée ci-dessus,
et, le cas échéant, à partir des acides carboxyliques correspondants, après hydrolyse et/ou activation préalables, on soumet ensuite à une amidation dans des solvants inertes des amines répondant à la formule générale (Va) 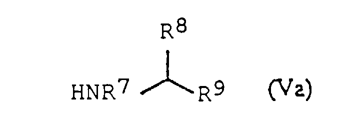 dans laquelle
R⁷, R⁸ et R⁹ ont la signification indiquée ci-dessus,
éventuellement en présence d'une base et/ou d'un adjuvant, par exemple d'un agent de déshydratation, et on sépare éventuellement les isomères que l'on fait réagir avec une base ou un acide correspondant dans le cas de la préparation des sels.

7. Médicament contenant au moins un dérivé d'acide phényl-cyclohexane-carboxylique substitué par un ou plusieurs composés hétéro-tricycliques selon les revendications 1 à 4.

8. Médicament selon la revendication 7, pour le traitement de l'hypertension artérielle et de l'athérosclérose.

9. Procédé pour la préparation d'un médicament selon la revendication 7, caractérisé en ce qu'on transforme les substances actives éventuellement à l'aide de substances auxiliaires et de substances de support appropriées en une forme d'application appropriée.

10. Utilisation de dérivés d'acides phényl-cyclohexane-carboxyliques substitués par un ou plusieurs composés hétéro-tricycliques selon les revendications 1 à 4, pour la préparation de médicaments.
